Europäisches Patentamt

European Patent Office

Office européen des brevets

(19)

(11) Numéro de publication : **0 113 287**
**B1**

(12) **FASCICULE DE BREVET EUROPÉEN**

(45) Date de publication du fascicule du brevet :
18.09.85

(21) Numéro de dépôt : 83402500.9

(22) Date de dépôt : 21.12.83

(51) Int. Cl.⁴ : **C 07 C 19/045, C 07 C 17/02**

(54) Procédé de préparation du dichloro-1,2 éthane.

(30) Priorité : 29.12.82 FR 8221970

(43) Date de publication de la demande :
11.07.84 Bulletin 84/28

(45) Mention de la délivrance du brevet :
18.09.85 Bulletin 85/38

(84) Etats contractants désignés :
AT BE CH DE GB IT LI LU NL SE

(56) Documents cités :
EP-A- 0 026 349
FR-A- 2 232 527

(73) Titulaire : **ATOCHEM**
**12/16, allée des Vosges**
**F-92400 Courbevoie (FR)**

(72) Inventeur : **Pellegrin, Daniel**
**12, Avenue Balard**
**F-04600 Saint-Auban (FR)**
Inventeur : **Correia, Yves**
**Les Lauzières**
**F-04160 Château-Arnoux (FR)**

(74) Mandataire : **Rochet, Michel et al**
**ATOCHEM Département Propriété Industrielle Cedex**
**22**
**F-92091 Paris la Défense (FR)**

Jouve, 18, rue St-Denis, 75001 Paris, France

## Description

La présente invention a pour objet un procédé de préparation de dichloro-1,2 éthane en phase liquide.

La demande de brevet européen n° 26349 décrit un procédé de préparation de dichloro-1,2 éthane par réaction d'addition de chlore à l'éthylène en phase liquide en présence de catalyseur à base d'acide de Lewis, procédé selon lequel la majeure partie de la chaleur de réaction est utilisée par la distillation du dichloro-1,2 éthane. Selon ce procédé, on effectue la réaction à une pression comprise entre 0,3 et 1,3 bar et à une température comprise entre 50 et 90 °C.

Ce procédé comporte un certain nombre de mesures permettant d'équilibrer le bilan thermique, notamment le débit de reflux, les conditions de refroidissement du produit réactionnel, l'introduction de chlore dans le produit réactionnel refroidi et le recyclage dans la chambre de réaction du produit réactionnel refroidi contenant du chlore dissous.

Compte tenu des conditions de pression et de température mentionnées ci-avant, le procédé décrit dans cette demande de brevet européen 26349 s'apparente en fait aux divers procédés en lit bouillant cités dans cette même demande 26349 et laisse entier le problème de la définition d'un réacteur dans lequel la température devrait être essentiellement homogène en tout point du réacteur alors que la concentration en chlore et éthylène devrait être essentiellement variable en tout point dudit réacteur.

L'invention se propose de fournir un procédé évitant les problèmes liés à l'utilisation d'un lit bouillant et évoqués ci-avant.

Un autre objet de l'invention est de proposer un procédé ne nécessitant pas l'emploi d'un réacteur de géométrie particulière.

L'invention fournit également un procédé selon lequel l'utilisation de l'énergie de réaction récupérée n'est pas limitée à la distillation du dichloro-1,2 éthane produit c'est-à-dire un procédé présentant une grande souplesse d'utilisation. Cette énergie de réaction est de l'ordre de 52,5 Kcal/mole lorsque l'on produit le dichloro-1,2 éthane sous forme liquide. Cette énergie est donc très excédentaire par rapport à la chaleur de vaporisation du dichloro-1,2 produit qui est de 7,65 Kcal/mole.

D'autres avantages de ce procédé apparaîtront à la lecture de ce qui suit.

L'invention a donc pour objet un procédé de préparation du dichloro-1,2 éthane pur par chloration de l'éthylène dans un dispositif comprenant un réacteur et une boucle de recirculation, en présence de catalyseur à base d'acide de Lewis et récupération de la chaleur de réaction, ledit procédé consistant à injecter du chlore et de l'éthylène dans un milieu réactionnel en circulation contenant du dichloro-1,2 éthane et le catalyseur, à soutirer et à rectifier le dichloro-1,2 éthane produit, ledit procédé étant caractérisé en ce que :

a) le milieu réactionnel est maintenu dans le réacteur de chloration et dans la boucle de recirculation à une température comprise entre 90 et 160 °C sous une pression empêchant la vaporisation du dichloro-1,2 éthane à la température choisie

b) la température du milieu réactionnel est maintenue à la valeur désirée par un échangeur générateur de vapeur permettant de récupérer la chaleur de réaction, la récupération et l'utilisation de cette chaleur de réaction étant totalement indépendantes de la rectification du dichloro-1,2 éthane.

L'indépendance totale de la récupération et de l'utilisation de la chaleur de réaction est une caractéristique importante du procédé conforme à l'invention. Bien évidemment, cette indépendance n'exclut pas l'utilisation de la chaleur de réaction dans une opération de distillation de dichloro-1,2 éthane mais ceci ne constitue qu'un emploi possible et non obligatoire des calories récupérées.

Dans ce qui suit, et en l'absence d'indication contraire, les expressions « poids du milieu réactionnel » ou « volume du milieu réactionnel » s'appliquent respectivement au poids et au volume du liquide contenu à la fois dans le réacteur et dans la boucle de recirculation.

Dans une forme de réalisation avantageuse, le procédé est mis en œuvre dans un dispositif comprenant :

— un réacteur de chloration proprement dit muni de condenseurs appropriés,

— une boucle de recirculation permettant l'homogénéisation du milieu réactionnel,

— les injecteurs de réactifs c'est-à-dire chlore et éthylène,

— une sortie de dichloro-1,2 éthane,

— un dispositif assurant un niveau constant dans le réacteur,

— un échangeur thermique disposé de préférence sur la boucle de recirculation, susceptible d'éliminer ou de récupérer la totalité de l'enthalpie de réaction, en assurant la vaporisation d'un fluide.

Cet ensemble est avantageusement complété par un circuit d'absorption des évents du réacteur (élimination des inertes, de chlore, azote, oxygène, CO, $CO_2$, d'acide chlorhydrique...).

Dans le procédé conforme à l'invention, on peut utiliser un catalyseur à base d'acide de Lewis pour ce type de réaction. D'une manière générale, on peut utiliser un chlorure métallique et notamment du chlorure ferrique, du chlorure d'antimoine ou du chlorure d'étain, ces produits étant cités à titre purement illustratif. La concentration du catalyseur peut varier de 0,005 à 0,5 % et de préférence entre 0,03 à 0,5 % en poids par rapport au poids du milieu réactionnel.

Le catalyseur peut être introduit directement dans le milieu réactionnel. Dans l'hypothèse de l'utilisation de $FeCl_3$ comme catalyseur, il peut également se former in situ par attaque des surfaces en fer

2

avec lesquelles le milieu réactionnel est en contact par le chlore en présence de traces d'eau présentes dans les réactifs.

Le rapport molaire des réactifs (chlore et éthylène) peut varier dans de larges limites. D'une manière générale, on préfère utiliser un léger excès d'éthylène par rapport à la quantité stœchiométrique nécessaire pour réagir avec le chlore, cet excès pouvant aller par exemple de 1 à 20 % c'est-à-dire un rapport molaire éthylène/chlore de 1,01 à 1,20. Les indications qui précèdent permettent entre autres d'éliminer des zones de concentration gazeuses à hauts risques tels que hydrogène + chlore.

Il va sans dire qu'on ne sortirait pas du cadre de l'invention en utilisant un léger excès de chlore.

Le chlore, qui est avantageusement introduit dans le milieu à l'état gazeux, renferme une faible quantité d'impuretés. Il est même souhaitable d'utiliser du chlore renfermant de l'oxygène, en général sous forme d'air, la concentration en oxygène pouvant être de l'ordre de 0,2 à 3 % et de préférence de 0,3 à 0,7 % en poids par rapport au poids du chlore.

Bien que le chlore puisse être introduit à tout endroit du réacteur ou de la boucle de recirculation, on l'injecte en général dans la boucle de recirculation, et de préférence en amont de la pompe de recirculation.

Cette façon de procéder permet de garantir une dissolution complète du chlore dans le dichloro-1,2 éthane en circulation, la dissolution complète des deux réactifs avant réaction (éthylène et chlore) étant une modalité particulièrement recommandée dans le procédé conforme à l'invention.

Pour les raisons qui précèdent, l'éthylène est injecté, également à l'état gazeux, à une certaine distance du point d'injection du chlore, c'est-à-dire dans le corps du réacteur lui-même lorsque le chlore est injecté dans la boucle de recirculation. Dans cette hypothèse, l'éthylène est avantageusement injecté à contre-courant du milieu réactionnel circulant.

La vitesse d'injection de l'éthylène est de préférence supérieure à 10 m/s et peut atteindre 100 m/s voire davantage. La vitesse d'injection du chlore est moins critique. De ce fait elle peut être du même ordre de grandeur que celle retenue pour l'injection de l'éthylène ou s'écarter de cette valeur, en restant de préférence à l'intérieur des limites 1 m/s à 100 m/s.

Le débit de recirculation du milieu réactionnel peut varier dans de larges limites. Ce débit est en général supérieur à 10 fois le volume du milieu réactionnel (contenu du réacteur et de la boucle de recirculation). De préférence, ce débit est compris entre 20 et 100 fois le volume du milieu réactionnel.

Dans le procédé conforme à l'invention, on maintient de préférence la totalité du milieu réactionnel à une température comprise entre 90 et 160 °C et plus particulièrement entre 100 et 130 °C, la pression dudit milieu étant au minimum de 3 à 8 ATA selon la température choisie, la pression maximum pouvant atteindre 15 ATA voire davantage, la condition essentielle dudit procédé résidant dans le fait que la totalité du milieu réactionnel reste liquide auxdites températures et pressions.

Ainsi qu'il a été précisé, la température du milieu réactionnel est maintenue à la valeur désirée et mentionnée ci-avant au moyen d'un échangeur générateur de vapeur. D'une manière générale, le fluide vaporisé est de l'eau mais on peut naturellement vaporiser d'autres fluides tels que par exemple les hydrocarbures chlorofluorés.

L'échangeur thermique permet de récupérer la totalité de l'enthalpie de la réaction à l'exception des pertes aux évents et des calories éventuellement utilisées pour le réchauffage des réactifs ce qui ne représente qu'au maximum 15 % de ladite enthalpie de réaction, ce qui signifie que dans ce procédé, au moins 85 % de cette enthalpie est récupérée et utilisable grâce à cet échangeur thermique.

Pour des raisons techniques évidentes, le soutirage est avantageusement effectué sur la boucle de recirculation bien qu'il soit possible de soutirer le dichloro-1,2 éthane sur le réacteur lui-même.

Le dichloro-1,2 éthane soutiré est débarrassé de son catalyseur par lavage, distillation flash, séparation du catalyseur, précipitation par un agent alcalin tel que $CaCO_3$ ou $Ca(OH)_2$ ou absorption sur charbon actif. Ces techniques sont décrites dans la littérature et ne constituent pas en elles-mêmes un objet de l'invention.

Selon une variante on effectue une vaporisation instantanée d'une partie du dichloro-1,2 éthane lors du passage du milieu réactionnel de la pression du réacteur à la pression atmosphérique (ou sous-atmosphérique), cette partie de dichloro-1,2 éthane anhydre et exempt de catalyseur étant amenée directement à la colonne de rectification.

Dans tous les cas le produit est rectifié dans un ensemble de colonnes. Un séchage peut précéder ce traitement.

On peut utiliser des agents desséchants tels que $CaCl_2$ ou faire appel à un tamis moléculaire. On donne cependant la préférence à un séchage par entraînement azéotropique de l'eau contenue dans le dichloro-1,2 éthane. Dans l'hypothèse d'un lavage à l'eau froide, après détente du dichloro-1,2 éthane, on peut alimenter la colonne de distillation azéotropique, s'il est fait appel à ce mode de séchage, au moyen de vapeur provenant de l'échangeur thermique mentionné précédemment.

La dernière étape du procédé conforme à l'invention réside dans la rectification, en une ou de préférence deux colonnes, du dichloro-1,2 éthane sec produit dans l'étape précédente. Cette rectification permet l'élimination des fractions légères (air, HCl, $Cl_2$, $H_2$, $C_2H_4$ et hydrocarbures chlorés) et des fractions lourdes.

A titre d'information, la productivité du procédé, exprimé en mole/l.h. (par rapport au volume du milieu réactionnel) peut aller de 1 à 5 et est de préférence compris entre 2 et 4.

Le procédé conforme à l'invention permet d'associer l'obtention d'un dichloro-1,2 éthane de haute pureté à une grande souplesse quant à l'utilisation de l'énergie thermique produite par la réaction de l'éthylène avec le chlore. En effet, la mise en œuvre de ce procédé dissocie totalement la réaction proprement dite, génératrice d'énergie de la rectification du dichloro-1,2 éthane. De plus, ce procédé convient particulièrement à la purification du dichloro-1,2 éthane par chloration des impuretés apparues à la suite de l'opération de cracking ou craquage conduisant au chlorure de vinyle monomère. En effet cette opération est génératrice d'impuretés nuisibles audit craking et qui se retrouvent dans le dichloro-1,2 éthane non cracké. Ces impuretés sont habituellement éliminées en associant des procédés de rectification, concentration et chloration. Elles peuvent être éliminées très efficacement par simple recyclage du dichloro-1,2 éthane non cracké les contenant, dans le milieu réactionnel du procédé de l'invention. L'échangeur thermique utilisé pour la récupération de la chaleur de réaction fournit de la vapeur basse pression, laquelle vapeur peut être transformée en vapeur haute pression par exemple par thermocompression et recevoir les usages les plus variés. Cette dissociation permet de concevoir aisément qu'un incident par exemple sur la section de rectification n'entraîne pas ipso facto l'arrêt de la totalité de la fabrication, une simple capacité de stockage permettant de pallier une déficience momentanée de ladite section de rectification. Par ailleurs, la lecture de l'exposé qui précède montre à l'évidence l'extrême simplicité de l'appareillage par comparaison avec les dispositifs nécessaires pour la réaction en lit bouillant.

Les exemples suivants illustrent l'invention.

## Exemple 1

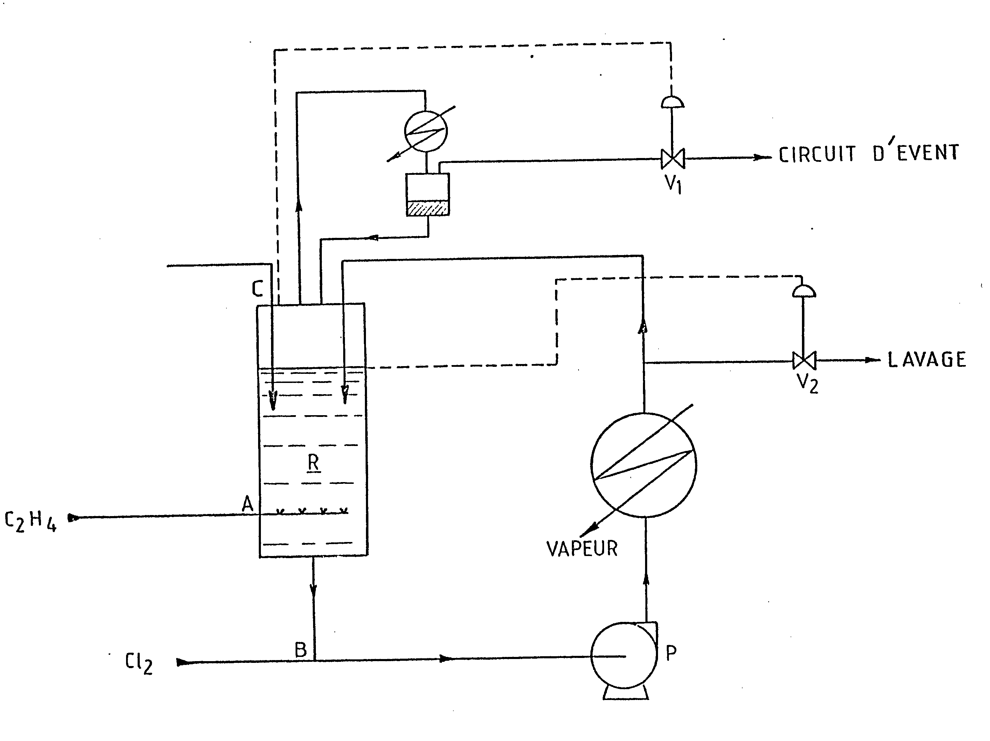

L'appareillage et le sens de circulation des flux sont schématisés sur la figure 1.

Dans le réacteur R en nickel maintenu à 120 °C sous 5 ATA et où la concentration en $FeCl_3$ est maintenue à 490 mg par litre de mélange réactionnel, on injecte par l'orifice (A) 15,75 moles/heure d'éthylène pur et par l'orifice (B) 15,34 moles/heure de chlore ayant la composition suivante :

$$Cl_2 = 97,8 \% \; ; \; N_2 = 1 \% \; ; \; O_2 = 0,5 \% \; ; \; CO_2 = 0,5 \% \; ; \; H_2 = 0,2 \%.$$

Le rapport du débit de recirculation de la pompe P au volume du milieu réactionnel est de 40. Le rapport molaire des débits d'éthylène et de chlore pur est de 1,05.

Le gaz qui s'échappe par la vanne $V_1$ vers le circuit d'évent est constitué par les inertes de la réaction, d'une partie de l'excès d'éthylène et des traces d'acide chlorhydrique.

Le débit du mélange réactionnel qui s'écoule par la vanne $V_2$ est de 14,8 moles/heures de dichloro-1,2 éthane brut dont la composition analytique en % poids est donnée dans le tableau 1.

Le rapport des moles de dichloro-1,2 éthane brut produites à l'heure au volume du milieu réactionnel est la mesure de la productivité de l'appareillage. Cette productivité est de 2 (en moles par heure et par litre).

A titre comparatif on donne dans le tableau 1 les résultats d'un essai réalisé à 60°.

## Exemple 2

On renouvelle l'essai de l'exemple 1, en conservant la même productivité mais en maintenant le milieu réactionnel à 140 °C au lieu de 120 °C. Les résultats sont rassemblés dans le tableau 1.

## Exemple 3

On renouvelle l'essai de l'exemple 1 en utilisant un rapport molaire $C_2H_4/Cl_2$ de 0,95.
Les résultats sont rassemblés dans le tableau 1.

## Exemple 4

Dans le réacteur décrit dans l'exemple 1, et avec les mêmes conditions opératoires, on introduit 15 moles/heure d'éthylène par l'orifice A, 15,54 moles/heure de chlore contenant toujours la même quantité d'inertes par l'orifice B et 200 cc/heure de dichloro-1,2 éthane contenant 22,36 g de trichloréthylène (0,17 mole) par l'orifice C.

La concentration en $FeCl_3$ est maintenue à 460 mg/h et la concentration stationnaire en chlore dissous s'établit à 0,043 % en poids.

Le dichloro-1,2 éthane brut qui s'écoule par la vanne V2 ne contient plus que 2 356 ppm de trichloréthylène, ce qui correspond à un taux de conversion du trichloréthylène de 76 %.

Le tableau 2 regroupe l'ensemble des caractéristiques et des résultats de cet essai.

Les 680 Kcal/h récupérées dans le procédé conforme à l'invention peuvent être utilisées pour la production de vapeur d'eau à 105° sous 1,2 bar. La pression peut être amenée à la valeur désirée (5 bars par exemple) par thermocompression.

4

La température de la vapeur atteint 152 °C sous 5 bars et cette vapeur fournit 760 Kcal/heure qui seront distribuées dans le réseau de l'usine.

Les résultats qui précèdent montrent à l'évidence que les calories récupérées sont largement suffisantes pour amener à 99,99 % le dichloro-1,2 éthane brut, de pureté comprise entre environ 95 et environ 99 %.

Dans l'exemple témoin, il n'y a pas de récupération de calories c'est-à-dire qu'il n'y a pas possibilité, sans apport extérieur d'énergie, de produire de la vapeur et/ou de rectifier le dichloro-1,2 éthane produit.

Tableau 1

| | 1 | | 2 | 3 |
| | Invention | Témoin | | |
|---|---|---|---|---|
| Température en °C | 120 | 60 | 140 | 120 |
| Pression en ATA | 5 | 1 | 6,5 | 5 |
| Rapport : $\dfrac{\text{Débit de recirculation}}{\text{Volume milieu réactionnel}}$ | 40 | 40 | 40 | 40 |
| Rapport molaire $\dfrac{C_2H_4}{Cl_2}$ | 1,05 | 1,05 | 1,05 | 0,95 |
| Productivité (moles/h.l.) | 2 | 2 | 2 | 2 |
| Concentration en $FeCl_3$ (mg/l) | 490 | 130 | 280 | 980 |
| Composition du dichloro-1,2 éthane brut obtenu en % (poids) : | | | | |
| Chlorure d'éthyle | 0,73 | 0,05 | 2,07 | 0,86 |
| Trichloro-1,1,2 éthane | 0,10 | 0,10 | 2,16 | 0,78 |
| Pentachloréthane | 0,27 | 0,01 | 0,18 | 0 |
| Autres impuretés | 0,17 | 0,11 | 0,16 | 0,08 |
| Pureté du dichloro-1,2 éthane | 98,73 % | 99,73 % | 95,43 % | 98,28 % |
| Récupération de calories (Kcal/heure) | 680 | 0 | 700 | 680 |

**0 113 287**

Tableau 2

| | Chloration simultanée du trichloréthylène |
|---|---|
| Température en °C | 120 |
| Pression en ATA | 5 |
| Rapport : $\dfrac{\text{Débit de recirculation}}{\text{Volume milieu réactionnel}}$ | 40 |
| Rapport molaire : $\dfrac{C_2H_4 + C_2HCl_3}{Cl_2}$ | 0,998 |
| Productivité (moles/h.1.) | 2 |
| Concentration en $FeCl_3$ (mg/1) | 460 |
| Composition du dichloro-1,2 éthane brut obtenu en % poids : | |
| Chlorure d'éthyle | 0,75 |
| Trichloro-1,1,2 éthane | 0,22 |
| Pentachloréthane | 0,99 |
| Trichloréthylène | 0,23 |
| Autres impuretés | 0,05 |
| Pureté du dichloro-1,2 éthane | 97,76 % |
| Récupération de calories (Kcal/heure) | 680 |

**Revendications**

1. Procédé permettant la préparation du dichloro-1,2 éthane pur par chloration de l'éthylène en phase liquide dans un dispositif comprenant une boucle de recirculation, en présence de catalyseur à base d'acide de Lewis et récupération de la chaleur de réaction, ledit procédé consistant à injecter du chlore et de l'éthylène dans un milieu réactionnel en circulation contenant du dichloro-1,2 éthane et le catalyseur, à soutirer et à rectifier le dichloro-1,2 éthane produit, ledit procédé étant caractérisé en ce que :

a) le milieu réactionnel est maintenu dans le réacteur de chloration et dans la boucle de recirculation à une température comprise entre 90 et 160 °C sous une pression empêchant la vaporisation du dichloro-1,2 éthane à la température choisie ;

b) la température du milieu réactionnel est maintenue à la valeur désirée par un échangeur générateur de vapeur permettant de récupérer la chaleur de réaction, la récupération et l'utilisation de cette chaleur de réaction étant totalement indépendantes de la rectification du dichloro-1,2 éthane.

2. Procédé selon la revendication 1, caractérisé en ce qu'au moins 85 % de l'enthalpie de la réaction est récupérée par l'échangeur générateur de vapeur.

3. Procédé selon l'une quelconque des revendications 1 ou 2, caractérisé en ce que la température du milieu réactionnel est comprise entre 100 et 130 °C.

4. Procédé selon l'une quelconque des revendications 1, 2 ou 3, caractérisé en ce que la pression du milieu réactionnel est au minimum de 3 à 8 ATA selon la température choisie.

5. Application du procédé selon l'une quelconque des revendications 1 à 4 à l'élimination des

6

**0 113 287**

impuretés présentes dans le dichloro-1,2 éthane de recyclage d'un cracking de dichloro-1,2 éthane destiné à la production de chlorure de vinyle monomère.

## Claims

1. Process permitting the preparation of pure 1,2-dichloroethane by chlorination of ethylene in liquid phase in a device incorporating a recirculation loop, in the presence of a catalyst based on a Lewis acid and recovery of the heat of reaction, the said process consisting in injecting chlorine and ethylene into a circulating reaction medium containing 1,2-dichloroethane and the catalyst, drawing off and rectifying the 1,2-dichloroethane produced, the said process being characterised in that :

a) the reaction medium is maintained in the chlorination reactor and in the recirculation loop at a temperature of between 90 and 160 °C under a pressure preventing the vaporization of 1,2-dichloroethane at the selected temperature ;

b) the temperature of the reaction medium is maintained at the required value by a steam-generator exchanger enabling the heat of reaction to be recovered, the recovery and the use of this heat of reaction being wholly independant of the rectification of 1,2-dichloroethane.

2. Process according to Claim 1, characterised in that at least 85 % of the enthalpy of reaction is recovered by the steam-generator exchanger.

3. Process according to either of Claims 1 or 2, characterised in that the temperature of the reaction medium is between 100 and 130 °C.

4. Process according to any one of Claims 1, 2 or 3, characterised in that the pressure of the reaction medium is at least 3 to 8 AA depending on the temperature selected.

5. Application of the process according to any one of Claims 1 to 4 to the removal of the impurities present in recycled 1,2-dichloroethane from cracking of 1,2-dichloroethane intended for the production of vinyl chloride monomer.

## Patentansprüche

1. Verfahren zur Herstellung von reinem Dichlor-1,2-ethan durch Chlorierung von Ethen in flüssiger Phase in einer Vorrichtung mit Rücklauf in Gegenwart eines Katalysators in Form einer Lewis-Säure und Rückgewinnung der Reaktionswärme, indem Chlor und Ethen in ein umlaufendes Reaktionsmedium enthaltend Dichlor-1,2-ethan und Katalysator, welches abgezogen wird, eingeleitet und das erhaltene Dichlor-1,2-ethan rektifiziert wird, dadurch gekennzeichnet, daß

a) das Reaktionsmedium im Chlorierungsreaktor und in der Rückleitung bei einer Temperatur zwischen 90 und 160 °C unter einem Druck gehalten wird, der ein Abdampfen des Dichlor-1,2-ethans bei der herrschenden Temperatur verhindert und

b) die Temperatur des Reaktionsmediums auf dem gewünschten Wert gehalten wird mit Hilfe eines dampferzeugenden Austauschers und Rückgewinnung der Reaktionswärme, wobei die Rückgewinnung und Verwertung der Reaktionswärme vollständig unabhängig ist von der Rektifizierung des Dichlor-1,2-ethans.

2. Verfahren nach Anspruch 1, dadurch gekennzeichnet, daß zumindest 85 % der Reaktions-Enthalpie in dem dampferzeugenden Austauscher rückgewonnen werden.

3. Verfahren nach Anspruch 1 oder 2, dadurch gekennzeichnet, daß die Temperatur des Reaktionsmediums zwischen 100 und 130 °C gehalten wird.

4. Verfahren nach Anspruch 1, 2 oder 3, dadurch gekennzeichnet, daß der Druck des Reaktionsmediums zumindest 3 bis 8 ata bei der herrschenden Temperatur beträgt.

5. Anwendung des Verfahrens nach einem der Ansprüche 1 bis 4 zur Entfernung der im Dichlor-1,2-ethan enthaltenen Verunreinigungen, die aus einem Kracken des Dichlor-1,2-ethan beim Rückleiten stammen, zur Herstellung des Ausgangsmaterials für die Produktion von monomerem Vinylchlorid.

7

CIRCUIT D'EVENT

$V_1$

LAVAGE

$V_2$

C

R

A

$C_2H_4$

B

$Cl_2$

VAPEUR

P

0 113 287